# EUROPEAN PATENT SPECIFICATION

(11) **EP 2 300 599 B1**
(45) Date of publication and mention of the grant of the patent: **01.03.2017**
(21) Application number: 09763477.8
(22) Date of filing: 09.06.2009
(51) Int. Cl.: A61K 35/12

(54) **AUGMENTATION OF CELL THERAPY EFFICACY INCLUDING TREATMENT WITH ALPHA 1-3 FUCOSLYTRANSFERASE**
STEIGERUNG DER WIRKSAMKEIT EINER ZELLTHERAPIE, EINSCHLIESSLICH BEHANDLUNG MIT ALPHA-1-3-FUCOSYLTRANSFERASE
AUGMENTATION DE L'EFFICACITÉ DE LA THÉRAPIE CELLULAIRE COMPRENANT LE TRAITEMENT AVEC L'ALPHA(1,3)FUCOSYLTRANSFÉRASE

(30) Priority: 09.06.2008 US 60084 P
(43) Date of publication of application: 30.03.2011
(73) Proprietor: Targazyme, Inc., Carlsbad, CA 92011 (US)
(72) Inventor: MILLER, Len, Carlsbad, CA 92009 (US); KOH, Lynnet, Carlsbad, CA 92008 (US); ICHIM, Thomas, E., San Diego, CA 92122 (US)
(74) Representative: Hallybone, Huw George
(86) International application number: PCT/US2009/046800
(87) International publication number: WO 2009/152187

(56) References cited:
- WO-A2-2004/094619
- WO-A2-2007/143204
- US-A1- 2004 247 574
- US-A1- 2005 048 036
- US-A1- 2006 228 340
- US-A1- 2007 190 023
- US-A1- 2008 044 383
- SACKSTEIN ROBERT ET AL: "Ex vivo glycan engineering of CD44 programs human multipotent mesenchymal stromal cell trafficking to bone", NATURE MEDICINE, NATURE PUBLISHING GROUP, NEW YORK, NY, US, vol. 14, no. 2, 1 February 2008 (2008-02-01), pages 181-187, XP002526137, ISSN: 1078-8956, DOI: 10.1038/NM1703 [retrieved on 2008-01-13]
- HIDALGO ANDRES ET AL: "Enforced fucosylation of neonatal CD34+ cells generates selectin ligands that enhance the initial interactions with microvessels but not homing to bone marrow", BLOOD, vol. 105, no. 2, 15 January 2005 (2005-01-15), pages 567-575, XP2526142, ISSN: 0006-4971
- SACKSTEIN R ET AL: "Mechanisms of lymphocyte migration to lymphoid tissues and inflamatory sites", CLINICAL IMMUNOLOGY NEWSLETTER, ELSEVIER, US, vol. 9, no. 11, 1 November 1988 (1988-11-01), pages 181-184, XP023714606, ISSN: 0197-1859, DOI: 10.1016/0197-1859(88)90073-8 [retrieved on 1988-11-01]
- XIA LIJUN ET AL: "Surface fucosylation of human cord blood cells augments binding to P-selectin and E-selectin and enhances engraftment in bone marrow", BLOOD, AMERICAN SOCIETY OF HEMATOLOGY, US, vol. 104, no. 10, 15 November 2004 (2004-11-15), pages 3091-3096, XP002429129, ISSN: 0006-4971, DOI: 10.1182/BLOOD-2004-02-0650

## Description

### BACKGROUND

### Field

The present embodiments relate generally to the field of cellular therapy. More specifically, some embodiments relate to methods of enhancing the natural process of cell migration through augmentation of specific glycosylation features on the surface of various cell types. More specifically, some embodiments relate to treatment of cells with fucosyltransferases in order to enhance the interaction between blood-borne stem cells, progenitor cells and endothelial cells facilitating entry into biological niches and tissues where they may function on a number of different levels for therapeutic and restorative intervention.

### Description of the Related Art

Cell therapy offers immense possibilities for treatment of a wide variety of medical conditions. Currently cell therapy is practiced in numerous embodiments, for example, bone marrow transplantation for treatment of hematopoietic and non-hematopoietic malignancies. The successful establishment of procedures for transplantation of donor cells into recipients whose function is malignant (leukemia) or altered (stroke, limb ischemia, etc), or insufficient (due to chemotherapy, radiotherapy, or congenital abnormality) constitutes a major medical breakthrough in the therapeutic management of these conditions.

One limiting factor of any cellular therapy is the need for blood-borne or directly injected cells to migrate to the targeted tissue in order to maximize their therapeutic potential. With regard to hematopoietic stem cells as a particular example, it is known that only a small percentage of these cells when administered systemically home to the bone marrow microenvironment. This migration is regulated in part by adhesive factors present on the luminal surface of endothelial cells which constitute the microvascular lining of the bone marrow and in part by chemotactic gradients secreted at a constant rate by bone marrow stromal cells. In addition, for the treatment of myocardial infarction or stroke, only a small fraction of injected stem cells actually home and enter the area of tissue damage. Thus, there exists a need to administer a high number of stem cells, sometimes prohibitively too high to be obtained in an autologous and even allogeneic setting.

### SUMMARY

The ability of cells such as leukocytes to interact with the endothelium has been known for decades. It has also been known that various glycosylation patterns are critical for cells such as leukocytes to "roll" on the endothelium prior to extravasation. What is desirable is the identification of novel methods that enhance cell trafficking and engraftment to areas of need in a simple and clinically applicable manner.

Accordingly, provided herein are methods of enhancing homing and engraftment of therapeutically-administered cells in a patient. It should be noted that the term "patient" is meant to broadly include any animal. For example, the animal can be a mammal, a bird, a fish, a reptile, a fish, an insect or any other animal. Some non-limiting examples of mammals may include humans and other primates, equines such as horses, bovines such as cows, mice, rats, rabbits, Guinea Pigs, pigs, and the like. It is also worth noting that the compositions and methods can be used with or applied to individual cells (for example *ex vivo* treatment or modification), to insect cells, etc. Also provided are cells that have been modified to enhance homing and engraftment. The embodiments provided herein are based in part on the surprising finding that by modification of molecules involved in the cell-endothelium interaction, it is possible to enhance the homing and subsequent efficacy of cellular therapy.

One embodiment provides a method of enhancing homing and engraftment of a therapeutically-administered cell in a patient in need of treatment with a cell population; providing cells that may have been contacted with an agent that modifies at least one surface molecule on the cells, resulting in a population of modified cells; and providing or administering the population of modified cells to a patient in need thereof. In certain aspects, the cell surface molecule may be modified so as to result in an alteration of cell charge.

In one embodiment, a method of enhancing homing and engraftment of a cell, may comprise providing one or more cells selected from stem cells, progenitor cells, neutrophils, macrophages and T-cells. The stem or progenitor cells may be embryonic stem cells, adult stem cells, expanded stem cells, placental stem cells, bone marrow stem cells, amniotic fluid stem cells, neuronal stem cells, cardiomyocyte stem cells, placental stem cells, endothelial progenitor cells, circulating and mobilized peripheral blood stem cells, muscle stem cells, germinal stem cells, adipose tissue derived stem cells, exfoliated teeth derived stem cells, hair follicle stem cells, dermal stem cells, parthenogenically derived stem cells, reprogrammed stem cells such as induced pluripotent stem cells or somatic nuclear transfer and side population stem cells. One or more cells may have been contacted with an agent that modifies at least one surface molecule on the cell that may result in enhanced selectin-mediated binding. This may result in a population of modified cells. These cells may be provided to animals. Such animals may include birds, reptiles, fish, insects, and mammals including but not limited to humans, equines such as horses, bovines such as cows, dogs, mice, rats, pigs, guinea pigs, rabbits and like.

In certain aspects of the above embodiments, the cell surface molecule may be modified by treatment with an enzyme and appropriate substrate(s) under conditions sufficient for causing an alteration of cell surface charge. In certain aspects, the enzyme may be a glycosidase, glycosyltransferase, a fucosyltransferase, a neuraminidase, an acetylglucosaminyltransferase, or any glycosyltransferase cpapble of increasing the number or affinity of cell surfave selectin binding components. In certain aspects, the enzyme may be alpha 1,3-fucosyltransferase I, alpha 1,3-fucosyltransferase III, alpha 1,3-fucosyltransferase IV, alpha 1,3-fucosyltransferase V, alpha 1,3-fucosyltransferase VI, alpha 1,3-fucosyltransferase VII, or alpha 1,3-fucosyltransferase IX.

In another aspect of the above embodiment, the cell may be treated with a reagent or reagents that link a binding unit to the cell surface. The binding unit may consist of a particle as well as a ligand of natural or non-natural sugars shown to possess binding affinity for receptors present on endotherial cells similar to that seen with natural sugars. The added binding unit may increase the functionalization of the cell.

In certain further aspects of the above embodiment, the cell may be treated with a single or plurality of molecules having ability to cause alpha 1-3 fucosylation of glycan determinants. In certain aspects, the molecule may be an alpha1-3 fucosyltransferase mixed together with a concentration of a fucose carrier under conditions sufficient to provide enhanced alpha 1-3 fucosylation of glycan determinants. In certain aspects, the fucose carrier may be guanosine diphosphate fucose. In certain aspects, the alpha 1-3 fucosyltransferase may be alpha 1-3 fucosyltransferase VI. In other aspects, the alpha 1-3 fucosyltransferase may be alpha 1-3 fucosyltransferase VII. In other aspects, the alpha 1-3 fucosyltransferase may be alpha 1-3 fucosyltransferase IV.

In certain aspects of the above embodiment, prior to the providing or administering, the population of modified cells has been further contacted for a period of time insufficient for cell division to occur with a CD26 peptidase inhibitor in an amount effective to inhibit CD26 peptidase activity and effective to increase the migratory response to CXCL12. PCT Application No. PCT/US2009/ , filed on June 9, 2009, entitled METHODS FOR ENHANCING CELL THERAPY EFFICACY INCLUDING TREATMENT WITH CD26 PEPTIDASE INHIBITORS, discloses and describes methods and compositions, any of which can be used with the technology of this application in any combination.

In certain aspects, prior to providing modified cells, a recipient may be contacted for a period of time and with sufficient dosing of a CD26 peptidase inhibitor in an amount effective to inhibit recipient CD26 peptidase activity effective to increase the migratory response of donor cells to chemotractant agents such as stromal cell-derived factor.

In certain aspects, the cell population may comprise or consist essentially of a population of stem cells, both embryonic and adult and expanded cell populations. In certain aspects, the stem cells may be embryonic stem cells, cord blood stem cells, placental stem cells, bone marrow stem cells, amniotic fluid stem cells, hematopoietic stem cells, mesenchymal stem cells, neuronal stem cells, cardiomyocyte stem cells, circulating and immobilized peripheral blood stem cells, endothelial progenitor cells, monocyte-derived stem cells, muscle stem cells, germinal stem cells, adipose tissue derived stem cells, exfoliated teeth derived stem cells, hair follicle stem cells, dermal stem cells, parthenogenically derived stem cells, reprogrammed stem cells such as induced pluripotent stem cells or somatic nuclear transfer and side population stem cells. In certain aspects, the embryonic stem cells may be totipotent. In certain aspects, the stem cell may be hematopoietic, mesenchymal, neural or cardiomyocyte stem cells. In certain aspects the hematopoietic stem cells may be further defined and differentiated as CD38-, lin- or ALDH-bright cells.

In certain aspects, the cell population may comprise or consist essentially of a population of committed progenitor cells or differentiated cells. In certain aspects, the cell population may be a mature blood cell population. In certain aspects, the mature blood cell may be neutrophils, macrophages, T-cells, activated T-cells, helper T cells, cytolytic T-cells, memory T-cells, regulatory T-cells or reprogrammed cells. In certain aspects, the T-cells may be from a heterogeneous population of T-cells.

In certain aspects, the patient in need of treatment with a cell population suffers from a malignant or non-malignant blood disorder such as an acute leukemia, a chronic leukemia, a myelodysplastic syndrome, a stem cell disorder, a myeloproliferative disorder, a lymphoproliferative disorder, a phagocyte disorder, a histiocytic disorder, a lysosomal storage disease, an age related disorder, an arterial or blood vessel or cardiovascular disorder, an enzyme deficiency disorder, a congenital immune system disorder, an inherited erythrocyte abnormality, an inherited platelet abnormality, a plasma cell disorder, a tumor or an autoimmune disease. In certain aspects, the patient in need of treatment with a cell population may suffer from peripheral arterial diseases, ischemic limb injury, diabetes, heart disease, bone disease, liver disease, muscular dystrophy, Alzheimer's disease, ALS, multiple sclerosis, Parkinson's disease, spinal cord injury, stroke or infertility.

In certain aspects, the population of modified cells may be administered intravenously, intraarterially, intramuscularly, subcutaneously, transdermally, intratracheally, intraperitoneally, intrathecally intracranially, intravitreally, or directly into the microvascular compartment of bone or into spinal fluid. In certain aspects, the population of modified cells may be administered in or proximal to a site of injury. In certain aspects, the homing and engraftment may take place within the bone marrow of the patient in need thereof.

In another embodiment, a composition may comprise an isolated population of cells modified for enhanced selectin-mediated binding. The isolated population of cells may be neutrophils, macrophages, T-cells, subpopulation of T-cells, or stem or progenitor cells selected from a group consisting of: embryonic stem cells, adult stem cells, expanded stem cells, placental stem cells, bone marrow stem cells, amniotic fluid stem cells, neuronal stem cells, cardiomyocyte stem cells, endothelial progenitor cells, circulating and mobilized peripheral blood stem cells, muscle stem cells, germinal stem cells, adipose tissue derived stem cells, exfoliated teeth derived stem cells, hair follicle stem cells, dermal stem cells, parthenogenically derived stem cells, or reprogrammed stem cells such as induced pluripotent stem cells or somatic nuclear transfer and side population stem cells and a pharmaceutically-acceptable carrier

In certain aspects, the isolated population may comprise a cell surface modification. In certain aspects, the cell surface molecule may be modified by treatment with an enzyme and appropriate substrate(s) under conditions sufficient for causing an alteration of cell surface charge.

In certain aspects, the enzyme is selected from a group comprising of: a glycosidase, a glycosyltransferase, a fucosyltransferase, a neuraminidase, and an acetylglucosaminyltransferase or any other glycotransferases capable of increasing cell surface selectin binding components. In certain aspects, the enzyme is selected from a group comprising of alpha 1,3-fucosyltransferase I, alpha 1,3-fucosyltransferase III, alpha 1,3-fucosyltransferase IV, alpha 1,3-fucosyltransferase V, alpha 1,3-fucosyltransferase VI, alpha 1,3-fucosyltransferase VII and alpha 1,3-fucosyltransferase IX.

In certain aspects, the cell may be treated with a single or plurality of molecules having ability to cause alpha 1-3 fucosylation of glycan determinants. In certain aspects, the molecule my be an alpha1-3 fucosyltransferase mixed together with a concentration of a fucose carrier under conditions sufficient to provide enhanced alpha 1-3 fucosylation of glycan determinants. In certain aspects, the fucose carrier may be guanosine diphosphate fucose. In certain aspects, the alpha 1-3 fucosyltransferase may be alpha 1-3 fucosyltransferase VI. In certain aspects, the alpha 1-3 fucosyltransferase may be alpha 1-3 fucosyltransferase VII. In certain aspects the alpha 1-3 fucosyltransferase may be alpha 1-3 fucosyltransferase IV. In certain aspects the molecule may be a non-naturally occurring enzyme having the ability to add a glycan determinant or a non-natural sugar that mimics the activity of fucose or other sugars that enhance the selectin binding process.

In certain aspects, the cell population may comprise or consist essentially of a population of stem cells both embryonic and adult. In certain aspects, the stem cells may be embryonic stem cells, cord blood stem cells, placental stem cells, bone marrow stem cells, amniotic fluid stem cells, hematopoietic stem cells, mesenchymal stem cells, neuronal stem cells, cardiomyocyte stem cells, circulating and mobilized peripheral blood stem cells, endothelial progenitor cells, monocyte-derived stem cells, muscle stem cells, germinal stem cells, adipose tissue derived stem cells, exfoliated teeth derived stem cells, hair follicle stem cells, dermal stem cells, parthenogenically derived stem cells, reprogrammed stem cells such as induced pluripotent stem cells or somatic nuclear transfer and side population stem cells. In certain aspects, the embryonic stem cells may be totipotent. In certain aspects, the stem cell may be hematopoietic, mesenchymal, neural or cardiomyocyte stem cells.

In certain aspects, the cell may be a mature blood cell. In certain aspects, the mature blood cell may be a neutrophil, macrophage, or T-cell. In certain aspects, the T-cells may be from a heterogeneous population of T-cells or from an ex vivo expanded cell population.

Another embodiment provides a method of enhancing homing and engraftment of a cell, comprising providing one or more cells selected from stem cells, progenitor cells, neutrophils, macrophages and T-cells. The stem or progenitor cells may be embryonic stem cells, adult stem cells, expanded stem cells, placental stem cells, bone marrow stem cells, amniotic fluid stem cells, neuronal stem cells, cardiomyocyte stem cells, endothelial progenitor cells, circulating and immobilized peripheral blood stem cells, muscle stem cells, germinal stem cells, adipose tissue derived stem cells, exfoliated teeth derived stem cells, hair follicle stem cells, dermal stem cells, parthenogenically derived stem cells, reprogrammed stem cells such as induced pluripotent stem cells or somatic nuclear transfer and side population stem cells. One or more cells may be contacted with an agent that modifies at least one surface molecule on the cell(s) to result in enhanced selectin-mediated binding, resulting in a population of modified cells.

Also provided herein is a method of fucosylation of cells so as to increase the ability of the cells to traffic, home and engraft into an area of biological need. The cells may be mature fully differentiated cells whose homing to specific targets is desired, such as islets, hepatocytes, or neutrophils or cells may be progenitor cells capable of differentiating into functional cells such as hepatic, renal, cardiac, or islet progenitors, or alternatively, the cells may be stem cells with multilineage differentiation ability such as embryonic stem cells, cord blood stem cells, placental stem cells, bone marrow stem cells, amniotic fluid stem cells, neuronal stem cells, circulating and mobilized peripheral blood stem cells, mesenchymal stem cells, endothelial stem cells, cardiomyocyte stem cells, germinal stem cells, committed endothelial progenitor cells, committed progenitor cells, adipose tissue derived stem cells, exfoliated teeth derived stem cells, hair follicle stem cells, dermal stem cells, parthenogenically derived stem cells, chemically, biologically, or electronically reprogrammed stem cells such as induced pluripotent stem cells or somatic nuclear transfer and side population of stem cells.

Other aspects relate to enhancing the ability of cells to modulate the immune system through enabling the cells to function with augmented efficiency at trafficking and homing. In another aspect, cells useful for immune therapy are "reprogrammed" ex vivo with endowment of distinct immunological properties. Surface modification may be performed before reprogramming, during reprogramming or after reprogramming. Reprogramming may be performed so as to increase immune stimulatory properties of the immune cells, or may be performed to allow the immune cells to suppress other immune cells. Reprogramming may be performed during expansion of cells, or to cells that have already been expanded.

In certain aspects, the cells can be fucosylated so as to enhance ability to home. Fucosylation may be performed on specific molecules present on the cells, or may be performed globally in a non-specific manner. Cells may be fucosylated through culture with an enzyme such as a fucosyltransferase capable of transferring fucose groups such as a fucosyltransferase. In certain aspects, the fucosyltransferase may be an alpha 1,3 fucosyltransferase. In another aspect the enzyme is selected from a group comprising of alpha 1,3-fucosyltransferase IX, alpha 1,3-fucosyltransferase III, alpha 1,3-fucosyltransferase IV, alpha 1,3-fucosyltransferase V, alpha 1,3-fucosyltransferase VI and alpha 1,3-fucosyltransferase VII. Appropriate culture conditions and substrates are also provided within the scope of the embodiments in order to allow proper fucosylation to occur. The conditions may include addition of substrates such as GDP-fucose or other similar compounds that provide a source of fucose.

In certain aspects, cells may be treated with a single or plurality of agents in order to augment expression of proteins involved in migration. Since the proteins involved in migration, when expressed de-novo, are not properly fucosylated, the addition of exogenous fucose groups increases ability of the de-novo expressed proteins to interact with endothelium and properly home. Specific molecules may include histone deacetylase inhibitors or DNA methyltransferase inhibitors.

### BRIEF DESCRIPTION OF THE DRAWINGS

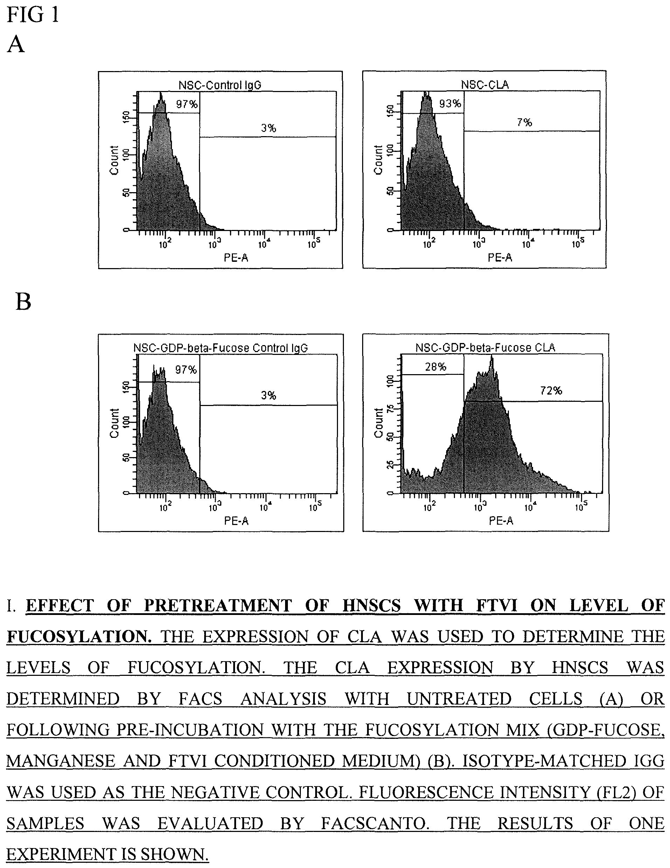

Figure 1 the effect of pretreatment of HNSCs with FTVI on level of fucosylation. The expression of CLA was used to determine the levels of fucosylation. The CLA expression by hNSCs was determined by FACS analysis with untreated cells (A) or following pre-incubation with the fucosylation mix (GDP-Fucose, manganese and FTVI conditioned medium) (B). Isotype-matched IgG was used as the negative control. Fluorescence intensity (FL2) of samples was evaluated by FACScanto. The results of one experiment are shown.

### DETAILED DESCRIPTION

Some embodiments relate generally to compositions for and methods of enhancing homing and engraftment of a therapeutically-administered cell in a patient. Also, some embodiments relate to cells that have been modified to enhance homing and engraftment. The embodiments provided herein are based in part on the surprising finding that by modification of molecules involved in the cell-endothelium interaction, it is possible to enhance the homing and subsequent efficacy of cellular therapy.

The process by which cells exit the systemic circulation and enter distinct biologically niches is a complex coordinated process that involves numerous molecules. The process of cellular exit is a bi-directional communication between the vascular endothelium cells and the circulating stem and progenitor cells. This process has been best characterized in the description of leukocyte exit from systemic circulation. An important family of molecules involved critically and initially in the process of cellular trafficking are the selectins. These molecules are type 1 transmembrane proteins that contain what is known as C-type lectin domains. Lectins are proteins that bind sugars. Most commonly known lectins include conconavalin-A and phytohemagglutinin. The C-type lectin domains on the selectins reside at the N-terminal of the selectins and interact with a wide array of glycoprotein ligands. Since the lectin domains bind sugar moieties, it is important that proper placement of sugars onto the proteins such that interaction of the selectins occurs. Since placement of sugars (glycosylation) usually occurs as a post-translational event, the mere genetic manipulation of cells is not sufficient usually to alter ability of cells to interact with selectins. The exception to this is, of course, genetic manipulation in the sense of transfecting cells with enzymes that are involved in the addition of sugars.

Provided herein are means of endowing enhanced trafficking/homing capabilities onto cells for use in cell therapy, said means consisting of modification of various glycosylation patterns on cells in order to augment ability to "roll", 'tether', and 'adhere' on the endothelium. The concept of cells rolling, tethering, and adhering on the endothelium is commonly known in the art and means of augmentation of this rolling process have been described strictly in the areas of hematopoietic cells, as well as tumor cells.

Previous studies with hematopoietc cells have demonstrated that it is possible to alter glycosylation and fucosylation patterns on the surface of cells by treating of cells with enzymes such as fucosyltransferases. In addition to establishing the ability to modify the surface of cells, the functional consequences of this modification has been documented. Specifically, several reports (Xia et al., Blood 2004 104:3091 - 3096; Hidalgo, et al., J. Clin. Invest. 2002 110:559 - 569, demonstrated that fucosylation of cord blood hematopoietic cells enhances binding of P and E selectin, enhances ex vivo binding to P and E-selectin-coated plates under physiological shear stress conditions and enhances homing and engraftment into bone marrow of NOD-SCID mice. (See also U.S. Patent No. 7,332,334 and U.S. Pub. No. 2006/0228340 to Xia and McEver). These findings in hematopoetic cells have been further described by others, including Sackstein et al. (U.S. 2003/0040607 and U.S. 2008/0044383).

However another report demonstrated that while early (minutes to hours following iv injection) adhesion of cord blood hematopoietic cells is increased after ex vivo fucosylation, no increase in bone marrow homing at 16 - 24 hrs was observed (Hidalgo and Frenette, Blood 2005 105:567 - 575).

It has been suggested by Sackstein et al. that mesenchymal stem cells, which do not express E-selectin ligands, can be glycosylated enzymatically in an effort to enhance migration of these cells to the bone marrow (Sackstein et al. Nature Medicine 2008 14:181- 187).

Thus, embodiments presented herein are based in part on the novel observation that fucosylation of ligands increases binding to tissue and can be used to enhance migration of various types of cells, enumerated herein, to an area of need. Specifically, some embodiments provided herein relate in part to the surprising finding that augmentation of the tethering and rolling process through various means is useful for enhancing functional capabilities of a wide variety of non-hematopoietic cells and stem cells. Accordingly, the methods and compositions provided herein may be useful for treatment of a wide variety of medical conditions which are amenable to cell therapy.

### Therapeutic Methods

In accordance with the above, provided herein are methods of enhancing homing and engraftment of a therapeutically-administered cell in a patient. Specifically, provided herein are methods of modification of sugar residues, both natural and non-natural, on the surface of cells used for cellular therapy so as to enhance their interaction with members of the selectin family, thereby enhancing trafficking of the cells administered systemically to an area of need.

Also provided are cells that have been modified to enhance homing and engraftment. The embodiments provided herein are based in part on the surprising finding that by modification of molecules involved in the cell-endothelium interaction, it is possible to enhance the homing and subsequent efficacy of cellular therapy.

Examples of cells include, without being limited thereto, neutrophils, macrophages and T-cells, wherein the stem or progenitor cells are selected from a group consisting of: embryonic stem cells, adult stem cells, expanded stem cells, placental stem cells, bone marrow stem cells, hematopoietic stem cells, mesenchymal stem cells, amniotic fluid stem cells, neuronal stem cells, cardiomyocyte stem cells, endothelial progenitor cells, circulating and mobilized peripheral blood stem cells, muscle stem cells, germinal stem cells, adipose tissue derived stem cells, exfoliated teeth derived stem cells, hair follicle stem cells, dermal stem cells, parthenogenically derived stem cells, reprogrammed stem cells such as induced pluripotent stem cells or somatic nuclear transfer and side population stem cells. In some aspects, one or more of the cell types mentioned can be specifically excluded from the methods or compositions described herein. As one example, in some aspects mesenchymal or hematopoietic stem cells can be excluded.

In one embodiment is provided a method of enhancing homing and engraftment of a therapeutically-administered cell in a patient comprising selecting a patient in need of treatment with a cell population; providing cells that have been contacted with an agent that modifies at least one surface molecule on the cells, resulting in a population of modified cells; and providing or administering the population of modified cells to a patient in need thereof. In certain aspects, the cell surface molecule is modified so as to result in an alteration of cell charge.

### Modification Enzymes

In certain aspects of the above embodiments, the cell surface molecule is modified by treatment with an enzyme and appropriate substrate(s) under conditions sufficient for causing an alteration of cell surface charge. Enyzmes which modify cell surface molecules are known in the art. Such enzymes include a purified glycosyltransferase polypeptide. Glycosyltransferase include for example, fucosyltransferase, galactosyltransferase, sialytransferase and N-acetylglucosaminotransferase. The fucosyltransferase can be, for example, an alpha 1,3 fucosyltransferase such as an alpha 1,3-fucosyltransferase I, alpha 1,3-fucosyltransferase III, alpha 1,3-fucosyltransferase IV, alpha 1,3-fucosyltransferase V, alpha 1,3-fucosyltransferase VI, alpha 1,3-fucosyltransferase VII, and alpha 1,3-fucosyltransferase IX. It should be noted that in some embodiments, one or more of the enzymes listed herein can be specifically excluded. For example, in some aspects, FTVI can be specifically excluded from the methods and compositions described herein.

In certain aspects, the cell surface molecule is modified in the presence of a sugar donor suitable for the specific glycosyltransferase. Sugar donors for glycosyltransferases are known in the art. For example, when the glycoslytransferase is a fucosyltransferase, the donor is GDP-fucose. Whereas, when the glycosyltransferase is a siayltransferase, the donor is CMP-sialic acid. In some instances the sugar can be a non-natural sugar added by a natural or modified glycosyltransferase.

The glycosyltransferases are biologically active. By biologically active is meant that the glycosyltransferases are capable of transferring a sugar molecule from a donor to acceptor. For example, the glycosyltransferase is capable of transferring 0.1, 0.2, 0.3, 0.4, 0.5, 1.0, 1.5, 2.0, 2.5, 5, 10 or more µmoles of sugar per minute at pH 6.5 at 37°C.

Physiologically acceptable solution is any solution that does not cause cell damage, e.g. death. For example, the viability of the cell or cell particle is at least 80%, 85%, 90%, 95%, 96%, 97%, 98%, 99% or more after treatment according to the methods presented herein. Suitable physiologically acceptable solutions include for example, Hank's Balanced Salt Solution (HBSS), Dulbecco's Modified Eagle Medium (DMEM), a Good's buffer (see N. E. Good, G. D. Winget, W. Winter, T N. Conolly, S. Izawa and R. M. M. Singh, Biochemistry 5, 467 (1966); N. E. Good, S. Izawa, Methods Enzymol. 24, 62 (1972) such as a HEPES buffer, a 2-Morpholinoethanesulfonic acid (MES) buffer, or phosphate buffered saline (PBS).

Thus, in certain embodiments provided herein, cells can be treated *ex vivo* with a kit that contains some or all of the agents, such as enzyme, buffer, cofactors and substrate necessary to achieve fucosylation of the cell surface glycoproteins that mediate adhesive interactions between the circulating cells following iv administration and endothelial cells at targeted tissue sites. In another embodiment provided herein, cells such as cord blood can be pretreated with the fucosylation kit prior to freezing or storage.

In certain aspects, the syn-anti, and or α-β substitution and orientation of the polysaccharide are modulated to provide the desired effect. Cell surface oligosaccharides are highly diversified in their structures and are associated with a variety of cell functions. In an inflammatory response, for example, neutrophils or leukocytes bind to injured tissues where the adhesion process occurs. This process has been found to be mediated by the tetrasaccharide sialyl Lewis X on neutrophiles or leukocytes and the receptor ELAM-1 (endothelial leukocyte adhesion molecule 1), a glycoprotein of the selectin family. Several sialyl lewis analogues and mimetics have been analyzed, in part, to understand affects of syn versus anti sugar conformers. Similar studies have examined fucosyl and galactosyl conformers, diastereomers, epimers and chiral analogues to examine adhesion and inhibitory properties. See Ichikawa Y et al; J. Am. Chem. Soc. 1992, 114, 9283-9298; Nelson, Richard M. et al. J. Clin. Invest. 1993, 1157-1166; Chun-Cheng Lin et al, J. Am. Chem. Soc. 1996, 118, 6826-6840; Clarke Julia L. J. Am. Chem. Soc. 1996, 118, 6826-6840; Chikara Ohyama, et al, The EMBO Journal Vol.18 No.6 pp.1516-1525, 1999; each of which is incorporated herein by reference in its entirety.

The introduction of fucose onto surface glycans of the cells of interest can be accomplished by enzymatic transfer from a donor substrate utilizing an alpha 1-3-fucosyltransferase (FT) by a process well known to someone skilled in the art. For this transfer to occur the cells at varying concentrations can be exposed to an incubation buffer containing a number of ingredients each of which can be optimized for efficient transfer of the fucose. The selection of buffer can come from a number of available buffers with Hanks balanced salt solution (HBSS) serving as the primary example. The substrate, guanosine diphosphate-fucose (GDP-fucose), at 1 mM can be mixed with the FT added at sufficient activity, expressed as Units/mL, to achieve maximal transfer of fucose to the cells of interest. In addition, MnCl2 at a final concentration of 0 - 10 mM can be added, if needed, depending on the cell population to further accelerate the enzymatic transfer reaction. The temperature and time of incubation can also be optimized for maximal transfer of fucose under practical application conditions with minimum toxicity to the cells of interest but is generally conducted at 37°C for 40 minutes.

Confirmation of fucosylated epitopes on the cells of interest as means of confirming maximal levels of fucosylation can be verified by Flow Cytometry utilizing agents and procedures well know to someone skilled in the art. For example, sialyl LewisX is a fucosylation epitope found on both P and E-selectins. By incubation of the FT-treated cells with anti-sLeX mAb HECA 452 (IgM) followed by treatment with FITC-conjugated fragment to the IgM, the sLeX epitopes on the cell surface can be visualized using standard Flow Cytometry procedures.

### Combination Treatment with CD26 Peptidase Inhibitors

In certain aspects of the above embodiment, prior to the administering, the population of modified cells has been further contacted for a period of time insufficient for cell division to occur with a CD26 peptidase inhibitor in an amount effective to inhibit CD26 peptidase activity and effective to increase the migratory response to CXCL12.

Exemplary methods of treating stem cells with CD26 (dipeptidylpeptidase) inhibitors are described in Christopherson et al. (U.S. Pub No. 2004/0247574, incorporated by reference in its entirety). In certain aspects, the CD26 inhibitor is selected from the group consisting of Diprotin A (Ile-Pro-Ile), Valine-Pyrrolidide, sitagliptin, vildagliptin, saxagliptin, alogliptin or any other class of compounds shown to exhibit potent inhibition of either purified, soluble or cell surface (CD26) dipeptidylpeptiase. PCT Application No. PCT/US2009/____, filed on June 9, 2009, entitled METHODS FOR ENHANCING CELL THERAPY EFFICACY INCLUDING TREATMENT WITH CD26 PEPTIDASE INHIBITORS, discloses and describes methods and compositions, any of which can be used with the technology of this application in any combination.

In certain aspects, the cell population is contacted with said CD26 inhibitor for about 5 minutes to about 12 hours or conditions suitable for sufficient inhibition of cell surface CD26 leading to an enhanced migratory response to chemotactic factors such as stromal cell-derived factor. In certain aspects, the cell population is contacted with said CD26 inhibitor for about 5 minutes to about 12 hours. In certain aspects, the cell population is contacted with said CD26 inhibitor for less than 6 hours. In certain aspects, the cell population is contacted with said CD26 inhibitor for less than 2 hours. In certain aspects, the cell population is contacted with said CD26 inhibitor for less than 1 hour.

In certain aspects, the inhibitor is administered in a concentration of less than about 1 nM, about 1 µM, about 5 µM, about 10 µM, about 50 µM, about 100 µM, about 1 mM or about 5 mM. In certain aspects, the inhibitor is administered in a concentration of no less than about 5 mM.

In certain aspects, at least 1 donor cell is treated. In selected embodiments, at least 1 x 10², 1 x 10³, 1 x 10⁴, 1 x 10⁵, 1 x 10⁶, 1 x 10⁷, 1 x 10⁸ donor cells per mL are treated.

In certain aspects the recipient can be treated with CD26 inhibitor simultaneous with injection of cell surface modified cells. In certain aspects the recipient can be treated with CD26 inhibitor prior to injection of cell surface modified cells. In certain aspects the recipient is pretreated with single or multiple doses of the CD26 inhibitor either simultaneous or prior to cell injection to achieve sustained inhibition of either or both administered cells and recipient CD26 activity leading to enhanced homing of administered cells.

### Therapeutic Cell Populations

Stem Cells. In certain aspects, the cell population comprises or consists essentially of a population of stem cells. In certain aspects, the stem cells are selected from a group consisting of: embryonic stem cells, cord blood stem cells, placental stem cells, bone marrow stem cells, amniotic fluid stem cells, hematopoietic stem cells, mesenchymal stem cells, neuronal stem cells, cardiomyocyte stem cells, circulating and immobilized peripheral blood stem cells, mesenchymal stem cells, germinal stem cells, adipose tissue derived stem cells, exfoliated teeth derived stem cells, hair follicle stem cells, dermal stem cells, parthenogenically derived stem cells, reprogrammed stem cells such as induced pluripotent stem cells or somatic nuclear transfer and side population stem cells or transdifferentiated cells. In certain aspects, the embryonic stem cells are totipotent.

As used herein, a "mesenchymal cell" means a cell forming a mesenchymal tissue, such as osteoblast, chondrocyte, myoblast, adipocyte, stroma cell, tendon cell, and the like, a mesenchymal stem cell capable of differentiating into these cells, and its premesenchymal stem cell. Mesenchymal cells generated during the embryo development, mesenchymal cells within an animal body, and mesenchymal cells differentiated and generated from pluripotent stem cells in vitro or in vivo are all encompassed in the term "mesenchymal cell."

As used herein, a "mesenchymal stem cell" means a mesenchymal cell possessing the ability of differentiating into mesenchymal cells of one or more types and the ability of self-replication. The mesenchymal stem cell differentiated from a pluripotent stem cell in vitro is positive for PDGFRα and negative for FLK1. Mesenchymal stem cells are able to differentiate into osteoblasts, chondrocytes, myoblasts, adipocytes, stroma cells, tendon cells, and the like, as with mesodermal cells.

As used herein, a "premesenchymal stem cell" means a mesenchymal cell possessing the ability of differentiating into mesenchymal stem cells of one or more types and the ability of self-replication. The premesenchymal stem cell differentiated from a pluripotent stem cell in vitro expresses Sox1, a neuroectodermal marker. The premesenchymal stem cell is able to differentiate into a mesenchymal stem cell which is PDGFRα-positive and FLK1-negative.

As used herein, a "neural stem cell" refers to a multipotent cell obtained from the central nervous system that can be caused to differentiate into cells that posses one or more biological activities of a neuronal cell type. Neural stem cells differentiate into neurons, astrocytes, and oligodendrocytes after plating onto substrates which stimulate adhesion and differentiation, for example poly-L-ornithine orlaminin. In addition, these multipotent CNS stem cells proliferate and expand in response to epidermal growth factor ("EGF") and basic fibroblast growth factor ("bFGF") and differentiate into neurons, astrocytes, oligodendrocytes, and muscle stem cells.

### Committed Progenitor Cells and Differentiated Cells.

In certain aspects, the cell population comprises or consists essentially of a population of committed progenitor cells or differentiated cells or transdifferentiated cells. In certain aspects, the cell population is a mature blood cell population. In certain aspects, the mature blood cell is selected from the group consisting of: neutrophils, macrophages and T-cells. In certain aspects, the T-cells are from a heterogeneous population of T-cells.

### Patients in Need of Treatment with Modified Cell Populations

In certain aspects, the patient in need of treatment with a cell population suffers from a condition selected from the group consisting of: an acute leukemia, a chronic leukemia, a myelodysplastic syndrome, a stem cell disorder, a myeloproliferative disorder, a lymphoproliferative disorder, a phagocyte disorder, a histiocytic disorder, a lysosomal storage disease, a congenital immune system disorder, an inherited erythrocyte abnormality, an inherited platelet abnormality, a plasma cell disorder, a tumor and an autoimmune disease. In certain aspects, the patient in need of treatment with a cell population suffers from a condition selected from the group consisting of: peripheral arterial diseases, ischemic limb injury, diabetes, heart disease, liver disease, bone disease, muscular dystrophy, Alzheimer's disease, ALS, multiple schlerosis, Parkinson's disease, spinal cord injury, stroke and infertility. Further examples of the above-described conditions are set forth in Table I below.

**Table I**

| **Acute Leukemias** | **Chronic Leukemias** |
|---|---|
| Acute Biphenotypic Leukemia | Chronic Lymphocytic Leukemia (CLL) |
| Acute Lymphocytic Leukemia (ALL) | Chronic Myelogenous Leukemia (CML) |
| Acute Myelogenous Leukemia (AML) | Juvenile Chronic Myelogenous Leukemia (JCML) |
| Acute Undifferentiated Leukemia | |
| | Juvenile Myelomonocytic Leukemia (JMML) |

| **Myelodysplastic Syndromes** | **Stem Cell Disorders** |
|---|---|
| Amyloidosis | Aplastic Anemia (Severe) |
| Chronic Myelomonocytic Leukemia (CMML) | Congenital Cytopenia |
| Refractory Anemia (RA) | Dyskeratosis Congenita |
| Refractory Anemia with Excess Blasts (RAEB) | Fanconi Anemia |
| Refractory Anemia with Excess Blasts in Transformation | Paroxysmal Nocturnal Hemoglobinuria (PNH) |
| (RAEB-T) | |
| Refractory Anemia with Ringed Sideroblasts (RARS) | |

| **Myeloproliferative Disorders** | **Lymphoproliferative Disorders** |
|---|---|
| Acute Myelofibrosis | Hodgkin's Disease |
| Agnogenic Myeloid Metaplasia (Myelofibrosis) | Non-Hodgkin's Lymphoma |
| Essential Thrombocythemia | Prolymphocytic Leukemia |
| Polycythemia Vera | |

| **Phagocyte Disorders** | **Histiocytic Disorders** |
|---|---|
| Chediak-Higashi Syndrome | Familial Erythrophagocytic |
| Chronic Granulomatous Disease | Lymphohistiocytosis |
| Neutrophil Actin Deficiency | Hemophagocytosis |
| Reticular Dysgenesis | Histiocytosis-X |
| | Langerhans' Cell Histiocytosis |

| **Lysosomal Storage Diseases** | **Congenital (Inherited) Immune System Disorders** |
|---|---|
| Adrenoleukodystrophy | |
| Alpha Mannosidosis | Absence of T and B Cells SCID |
| Gaucher's Disease | Absence of T Cells, Normal B Cell SCID |
| Hunter's Syndrome (MPS-II) | Ataxia-Telangiectasia |
| Hurler's Syndrome (MPS-IH) | Bare Lymphocyte Syndrome |
| Krabbe Disease | Common Variable Immunodeficiency |
| Maroteaux-Lamy Syndrome (MPS-VI) | DiGeorge Syndrome |
| Metachromatic Leukodystrophy | Kostmann Syndrome |
| Morquio Syndrome (MPS-IV) | Leukocyte Adhesion Deficiency |
| Mucolipidosis II (I-cell Disease) | Omenn's Syndrome |
| Mucopolysaccharidoses (MPS) | Severe Combined Immunodeficiency (SCID) |
| Niemann-Pick Disease | SCID with Adenosine Deaminase Deficiency |
| Sanfilippo Syndrome (MPS-III) | Wiskott-Aldrich Syndrome |
| Scheie Syndrome (MPS-IS) | X-Linked Lymphoproliferative Disorder |
| Sly Syndrome, Beta-Glucuronidase Deficiency (MPS-VII) | |
| Wolman Disease | |

| **Inherited Erythrocyte Abnormalities** | **Other Inherited Disorders** |
|---|---|
| Beta Thalassemia Major | Cartilage-Hair Hypoplasia |
| Blackfan-Diamond Anemia | Ceroid Lipofuscinosis |
| Pure Red Cell Aplasia | Congenital Erythropoietic Porphyria |
| Sickle Cell Disease | Glanzmann Thrombasthenia |
| | Lesch-Nyhan Syndrome |
| | Osteopetrosis |
| | Sandhoff Disease |

| **Inherited Platelet Abnormalities** | **Plasma Cell Disorders** |
|---|---|
| Amegakaryocytosis / Congenital | Multiple Myeloma |
| Thrombocytopenia | Plasma Cell Leukemia |
| | Waldenstrom's Macroglobulinemia |

| **Other Malignancies** | **Autoimmune Diseases** |
|---|---|
| Brain Tumors | Multiple Sclerosis |
| Ewing Sarcoma | Rheumatoid Arthritis |
| Neuroblastoma | Systemic Lupus Erythematosus |
| Ovarian Cancer | Diabetes Mellitus |
| Renal Cell Carcinoma | Inflammatory Bowel Diseases |
| Small-Cell Lung Cancer | |
| Testicular Cancer | |

| **Other Applications** | |
|---|---|
| Bone Marrow Transplants | |
| Heart Disease (myocardial infarction), either alone or in | |
| combination with enhancing agents such as erythropoietin | |
| Liver Disease | |
| Muscular Dystrophy | |
| Alzheimer's Disease | |
| Parkinson's Disease | |
| Spinal Cord Injury | |
| Stroke, either alone or in combination with enhancing agents | |
| such as erythropoietin | |
| Peripheral Vascular Disease | |
| Head trauma | |
| Ex vivo and In vivo expanded stem and progenitor cell populations | |
| In vitro fertilization application and enhancement | |
| Hematopoietic Rescue Situations (Intense Chemo/Radiation) | |
| Stem cells and progenitor cells derived from various tissues | |
| sources | |
| Application in humans and animals | |
| Limb regeneration, alone or in combination with enhancing | |
| agents | |

Cellular therapy is also desirable for treatment of diseases in which the immune system is sought to be enhanced. One particular form of cell therapy involves the expansion of T cells that possess specificity for a distinct antigen, for example a tumor antigen. In other types of cell therapy, T cells are generated, and reprogrammed ex vivo for ability to kill a plurality of cells that express a plurality of markers. Examples of such cell therapy include expansion of autologous T cells with IL-2, stimulation with tumor cell lysates, and reintroduction of said cells into the patient.

On the other hand, cell therapy may be performed in situations where suppression of an immune response is desired. In such situations expansion of cells such as CD4+ CD25+ regulatory T cells is desirable since these cells are capable of inhibiting immune responses in an antigen-specific manner. Methods for expansion of these cells are commonly known and include use of cytokines such as TGF-b.

One issue in bone marrow homing is that the receptors on endothelial cells for the glycosylated ligands of circulating cells are constitutively expressed. These receptors, such as P and E selectins, induce numerous activities after interacting with cells, including causing apoptosis or proliferative arrest (Winkler et al., Blood 2004 103: 1685 - 1692). Accordingly, the administration of hematopoietic cells and their subsequent homing to the bone marrow is dependent on molecules that are consitutively expressed.

For the purpose of a broader application of this approach for regenerative medicine in which cells are administered for non-hematopoietic purposes, the trafficking/homing of cells to the targeted location is much more complex and involves ligands that are not constitutively expressed, but expressed as a result of inflammation or tissue damage. For example, administration of stem cells for the purpose of treating myocardial infarction depends on homing of these cells to areas bathed in cytokine locally released which not only induces expression of E selectin and P selectin on the endothelium but also mediates chemoattraction to the site. This tissue localized upregulation of receptors and chemoattractant agents allows for homing of stem cells into areas of injury.

### Routes of Administration

Administration of the modified cells is performed in agreement with standard practices that are known to one skilled in the art. Several embodiments are possible. For example, routes of administration may include parenteral, e.g., intravenous, intradermal, microvascular bed of bone marrow, subcutaneous, oral (e.g., ingestion or inhalation), transdermal (topical), transmucosal, and rectal administration. In certain particular aspects, the population of modified cells is administered from a route selected from a group consisting of: intravenously, intraarterially, intramuscularly, subcutaneously, transdermally, intratracheally, intraperitoneally, intravitreally, via direct injection, into bone compartments or into spinal fluid. In some aspects the cells, compositions or other materials can be used with a scaffolding support. In certain aspects, the population of modified cells is administered in or proximal to a site of injury. In certain aspects, the homing and engraftment takes place within the bone marrow of the patient in need thereof. In certain aspects the cells are administered by multiple routes and sites either simultaneously or sequentially.

In another embodiment, the methods, compositions, cells and other materials can be useful for enhancing functional capabilities in a wide variety of not only hematological disorders but also non-hematological disorders. Specifically, the methods, compositions, cells and other materials may be useful for the treatment of medical conditions which are amenable to cell therapy. More specifically, the methods, compositions, cells and other materials may be useful for the treatment of acute leukemias, chronic leukemias, myelodysplastic syndromes, stem cell disorders, myeloproliferative disorders, lymphoproliferative disorders, phagocyte disorders, histiocytic disorders, lysosomal storage diseases, congenital immune system disorders, inherited erythrocyte abnormalities, other inherited disorders, inherited platelet abnormalities, plasma cell disorder, various malignancies such as brain tumors or Ewing sarcoma, Autoimmune Diseases, and other applications such as bone marrow transplants, diabetes, heart disease, liver disease, hematopoietic rescue situations following intense chemo/radiation, limb ischemia and limb regeneration (including cartilage regeneration, skin regeneration, blood vessel regeneration, etc), cartilage regeneration, skin regeneration, blood vessel regeneration, etc.

Furthermore, presented herein is the finding that the general increased adhesion of cells that have been fucosylated *ex vivo* can be utilized for augmented binding to localized niche areas in absence of chemotactic gradient such as in the context of portal vein injection or pulmonary artery injection.

For P-selectin binding cells can be incubated with anti-CD34+-PE and with P-selectin isolated from human platelets. P-selectin binding can be detected with FITC-labeled S12, a non-blocking mAb to human P-selectin. For E-selectin binding cells can be incubated with E-selectin/IgM after Fc receptor blocking. E-selectin can then be detected with FITC-labeled goat anti-human IgM polyclonal antibodies. Visualization of binding can be achieved using FACS analysis. Incubation for both P and E-selectin can be carried out at 4oC for 20 min.

To confirm a functional consequence of fucosylation following treatment with fucosyltransferase the cells can be examined for adhesion to either E-selectin or P-selectin under physiological shear forces using an in vitro flow chamber rolling assay system. P-selectin isolated from human platelets can be immobilized on plates in a parallel-plate flow chamber. A P-selectin site density of about 145 sites/µm2 can be used and measured by binding of 125I-labeled anti-P-selectin mAb S12. For E-selectin soluble human E-selectin can also be immobilized on plates in a parallel-plate flow chamber at a density of 200 sites/µm2 as measured by binding of 125I-labeled anti-human E-selectin mAb ES1. Sham-treated or FTVI-treated cells (in Hanks' balanced salt solution and 0.5% human albumin) can be perfused over P- or E-selectin coated plates at a wall shear stress of 1 dyn/cm2. The accumulated number of rolling cells can be measured with the aid of a videomicroscopy system coupled to an image analysis system. Specificity of interaction of cells with the coated plates can be confirmed with the inclusion of specific inhibitors to the binding and examination of rolling on plates coated only with human serum albumin.

### EXAMPLES

### EXAMPLE 1

### PARAMETERS FOR MAXIMAL FTVI ACTIVITY IN CORD BLOOD

Enzymatic-mediated fucosylation (α 1-3-linked fucose addition to cell-surface glycans) has shown both phenotypic and functional changes in MNC and CD34+ cell populations.

These *in vitro* studies are structured to examine the various components integral to the enzymatic-mediated fucosylation using a cell preparation procedure routinely practiced in the clinic. A frozen thawed human cord blood mononuclear cell population is washed by a procedure that involves a 1 to 10 dilution with chilled 10% Dextran-40/5% HSA solution, placing this diluted solution in a pre-cooled (2- 6°C) centrifuge for 5 - 10 min followed by mild centrifugation at approximately 550 g for 20 min. The supernatant is discarded while the pellet is resuspended in Hank's balanced salt solution (HBSS) containing 1% HSA at a target cell concentration ranging from 0.5 x 10⁶ to 1 x 10⁹ per ml. This cell population suspended in fucosyltransferase VI (FTVI) reaction media constitutes the core preparation used to examine a range of various parameters in order to identify the optimal conditions for maximal activity of FTVI within this cell population. The parameters examined are Mn++ (over final concentration ranging from 0.0 to 10 mM), GDP-fucose (over final concentration ranging from 0.3 to 10 mM), time course analysis with varying periods of incubation (ranging from 15 min to 60 min), FTVI (over a 20 fold range of enzyme concentration), temperature [10°C, 25°C (considered as room temperature) and 37°C] and cell concentration (over final concentration ranging from 0.5 x 10⁶ to 1 x 10⁹ per ml). Additional activities within these optimization efforts include assessing the stability of the fucosylated product with an examination of HECA-452 binding at 30, 60, 120, 180 and 240 min post termination of reaction and assessing the extent of fucosylation of additional cell populations. The cell preparation in reaction mix is incubated for 30 min (except for time course study) with occasional gently mixing. The solutions are then diluted with cold HBSS 1% HSA, filtered through 70 micron cell screen and subjected to chilled centrifugation for 12 min. The supernatant is discarded, pellet loosened and resuspended for either injection or analysis by FACS using a procedure that is familiar to someone skilled in the art. For FACS analysis, aliquots of the cells, at approximately 5 x 10⁵, are centrifuged at 500 x g for 13 minutes at 4°C. The supernatant is discarded, pellet loosened for the addition of flow stain cocktail containing the appropriate stain(s) or control cocktail. The mixture is stored in the dark for 30 - 40 min with occasional mixing. Each tube is diluted with 3 ml of cold flow wash buffer followed by centrifugation at 500 x g for 12 min at 4°C. The supernatant is discarded, pellet loosened with the addition of approximately 200 µl of cold flow buffer or flow fix buffer. Aliquots of each sample are examined by FACS for percentage of double positives for CD34+ vs HECA-452 which is the primary outcome measure for determining maximal expression of FTVI activity in the cell mix. Also generated as an outcome measure for assessment of enzyme activity is the mean fluorescence intensity

### EXAMPLE 2

### ENHANCED ENGRAFTMENT IN THE BONE MARROW USING A COMBINATION APPROACH CONSISTING OF MAXIMAL FUCOSYLATION OF CELL PREPARATION PLUS EXPOSUE TO A CD26 INHIBITOR

First, maximal fucosylation of cells *in vitro* is accomplished. To accomplish this, washed mononuclear cells (MNCs) are resuspended in Hank's Balanced Salt Solution (HBSS) at a concentration of 0.5 x 10⁶ - 1 X 10⁹ per ml and then incubated with a fucosylation mix consisting at final concentration of 5 mM GDP-fucose, purified human recombinant α1-3 fucosyltransferase VI at a predetermined Units per ml (for maximal fucosylation), and 1 - 10 mM MnCL₂ in HBSS. This mix is incubated for 30 min at 37°C or room temperature in a humidified atmosphere containing 5% CO₂. The period of incubation could take longer or shorter depending on the incubation temperature chosen. Following completion of the fucosylation and to achieve inhibition of CD26 (dipeptidylpeptidase, DPPIV) as part of the combination approach, this preparation of fucosylated mononuclear cells (MNCs) is either washed first or directly incubated with a potent DPPIV inhibitor for 5 - 15 min at room temperature at a concentration sufficient to achieve complete or nearly complete inhibition of DPPIV. Following incubation the cell suspension is volume adjusted with HBSS or another clinically compatible solution to obtain the appropriate concentration of cells in preparation for iv injection. As an alternative scenario for a combination approach, the patients are subjected to systemic administration of the DPPIV inhibitor at a dose sufficient to achieve a sustained level in the body for significant inhibition of bone marrow and circulating plasma DPPIV activity. Subsequent to systemic pretreatment, MNCs exposed to conditions for maximal fucosylation are then injected into the patient. The DPPIV inhibitor can be added simultaneous with the injection of the fucosylated cell preparation or shortly before. The patients are prepared for this combined approach by subjecting them to conditions for myeloablation or even partial myeloablation (mini) prior to injection of the fucosylated and inhibitor treated cells. The rate of recovery and extent of chimerism is assessed with an examination of serially collected blood samples in addition to an examination of cells obtained from the bone marrow. A multilineage analysis of the rate of recovery and extent of engraftment and chimerism is accomplished using cell surface markers specific for cell types in addition to an examination of mature hematopoietic cells in the blood stream. These markers and cells are detected using preparation techniques and FACS analysis procedures that are familiar to one skilled in the art.

### EXAMPLE 3

### EX VIVO FUCOSYLATION OF MESENCHYMAL STEM CELLS

The introduction of fucose onto surface glycans of mesenchymal stem cells (MSC) is accomplished by enzymatic transfer from a donor substrate utilizing an alpha 1-3-fucosyltransferase (FT). For this transfer to occur the cells at varying concentrations are exposed to an incubation buffer containing a number of ingredients each of which has been optimized for efficient transfer of the fucose, and performed in Hanks balanced salt solution (HBSS). The substrate, guanosine diphosphate-fucose (GDP-fucose), at 1 mM is mixed with the FT added at sufficient activity, in order to achieve maximal transfer of fucose to MSCs. In addition, MnCl₂ at a final concentration of 0 - 10 mM is added, as needed, to further accelerate the enzymatic transfer reaction. The incubation is performed at 37°C for 40 minutes with minimum toxicity to the cells.

Confirmation of fucosylated epitopes on the cells of interest as means of confirming maximal levels of fucosylation is verified by Flow Cytometry in order to detect sialyl LewisX (sLeX), a fucosylation epitope found on both P and E-selectins. The FT-treated cells are incubated with anti-sLeX mAb HECA 452 (IgM), followed by treatment with FITC-conjugated fragment to the IgM. Finally, the sLeX epitopes on the cell surface are visualized using standard Flow Cytometry procedures.

To measure P-selectin binding, cells are incubated with anti-CD34+-PE and with P-selectin isolated from human platelets. P-selectin binding is detected with FITC-labeled S12, a non-blocking mAb to human P-selectin. To measure E-selectin binding, cells are incubated with E-selectin/IgM after Fc receptor blocking. E-selectin is then detected with FITC-labeled goat anti-human IgM polyclonal antibodies. Visualization of binding is achieved using FACS analysis. Incubation for both P and E-selectin is carried out at 4°C for 20 min.

To confirm a functional consequence of fucosylation following treatment with FT the cells are examined for adhesion to either E-selectin or P-selectin under physiological shear forces using an in vitro flow chamber rolling assay system. P-selectin isolated from human platelets is immobilized on plates in a parallel-plate flow chamber. A P-selectin site density of about 145 sites/µm² is used and measured by binding of ¹²⁵I-labeled anti-P-selectin mAb S12. For E-selectin soluble human E-selectin is also immobilized on plates in a parallel-plate flow chamber at a density of 200 sites/µm² as measured by binding of ¹²⁵I-labeled anti-human E-selectin mAb ES1. Sham-treated or FTVI-treated cells (in Hanks' balanced salt solution and 0.5% human albumin) is perfused over P- or E-selectin coated plates at a wall shear stress of 1 dyn/cm². The accumulated number of rolling cells is measured with the aid of a videomicroscopy system coupled to an image analysis system. Specificity of interaction of cells with the coated plates is then confirmed with the inclusion of specific inhibitors to the binding and examination of rolling on plates coated only with human serum albumin.

### EXAMPLE 4

### ADMINISTRATION OF MODIFIED STEM CELLS TO BONE MARROW TRANSPLANT PATIENTS

Patients in need of a bone marrow transplant are subjected to either myeloablative or non-myeloablative conditions. Stem cells obtained from one of a number of different sources are incubated *ex vivo* with fucosyltransferase + GDP-fucose at sufficient concentrations and for a sufficient period of time to result in maximal formation of fucosylated product, such as sialyl Lewis X, on the cell surface. Following treatment, the cell preparation is washed or directly injected into the patient. Effectiveness of this application in the patient is determined with accelerated appearance over time of neutrophils and platelets in the blood stream compared to patients injected with control untreated stem cells.

### EXAMPLE 5

### MODIFICATION OF NEURAL STEM CELLS

A cell population consisting of neural stem cells is treated with conditions so as to endow increased surface ligands for enhanced interaction with endothelium. Cells are modified with the addition of alpha 1-3-linked fucose to cell-surface glycans by ex vivo treatment of cells with the enzyme alpha 1-2 fucosyltransferase VI. Specifically, cells are treated with 1 mM GDP fucose, 20 mU/mL {alpha}1-3 fucosyltransferase VI, and 10 mM MnCl2 in 0.5 mL HBSS containing 1% human serum albumin (HSA) for 30 minutes at 37°C in a humidified atmosphere containing 5% CO2 under conditions that cause minimum toxicity to CD34+ cells as tested by propidium iodide staining measured by flow cytometry. Other modifications of this treatment procedure may be performed based on the knowledge of one skilled in the art. Said treated neural stem cells are subsequently assessed for fucosylation using flow cytometric methodology. The cells are placed on ice for 5 min followed by washing with PBS (1 ml). To detect the presence of new fucosylated units on cell surface the cell preparation is treated with the 1^{st} antibody, anti-CLA 1:200 dilution in blocking buffer (400ul), and then incubated for one hour at room temp or overnight at 4°. Cells are then washed three times with PBS. The secondary antibody (anti-rat-IgM-PE, 1:200) in blocking buffer (400ul) is then added. The preparation is incubated for 2 hrs at room temperature then rinsed with PBS. The result of this treatment and analysis is shown in Fig 1.

### EXAMPLE 6

### MODIFICATION OF IMMUNE MODULATORY CELLS

A cell population consisting of cells with immune modulatory potential are treated with certain conditions so as to endow increased surface ligands for endothelium. Cells are modified with alpha 1-3-linked fucose to cell-surface glycans by treatment of cells with the enzyme alpha 1-2 fucosyltransferase VI. Specifically, cells are diluted to a concentration of 10(7) per ml and treated with 1 mM GDP fucose (EMD Biosciences, San Diego, CA), 20 mU/mL {alpha} 1-3 fucosyltransferase VI (FTVI; EMD Biosciences), and 10 mM MnCl2 in 0.5 mL HBSS containing 1% human serum albumin (HSA) for 30 minutes at 37°C in a humidified atmosphere containing 5% CO2 under conditions that cause minimum toxicity to CD34+ cells as tested by propidium iodide staining measured by flow cytometry. Other modifications of this treatment procedure may be performed based on the knowledge of one skilled in the art. Said treated immune cells are subsequently assessed for fucosylation status using either a flow cytometric methodology (assessment of HECA-453 binding) or functional methodology (assessment of rolling on endothelium). Cells are subsequently administered to a patient for immune modulation.

### EXAMPLE 7

### AUGMENTING EFFICACY OF TUMOR INFILTRATING LYMPHOCYTES AFTER EX VIVO EXPANSION

Tumor infiltrating lymphocytes are collected as described by Zhou et al The Journal of Immunology, 2005, 175: 7046-7052. Briefly, explants of small (2 mm3) tumor fragments or 1 x 106 viable cells of tumor tissue digests are used to initiate TIL culture in 2 ml of RPMI 1640-based medium (Invitrogen Life Technologies) containing 10% human serum and 6000 IU/ml IL-2 (Chiron). After 2-4 wk of culture, usually several million TIL cells are obtained and screened by IFN secretion assay for recognition of tumor cells. Antitumor TIL cultures were further expanded in AIM V medium (Invitrogen Life Technologies) supplemented with irradiated allogeneic feeder cells, anti-CD3 Ab (Ortho Biotech), and 6000 IU/ml IL-2. This expansion protocol typically resulted in 1000-fold expansions of cells by the time of administration 14-15 days after initiation of the expansions. Subsequent to expansion cells are harvested, centrifuged, diluted to a concentration of 10(7) per ml and treated with 1 mM GDP fucose (EMD Biosciences, San Diego, CA), 20 mU/mL alpha 1-3 fucosyltransferase VI (FTVI; EMD Biosciences), and 10 mM MnCl2 in 0.5 mL HBSS containing 1% human serum albumin (HSA) for 30 minutes at 37°C in a humidified atmosphere containing 5% CO2 under conditions that cause minimum toxicity to cells as tested by propidium iodide staining measured by flow cytometry. Cells are administered on a weekly basis at a concentration of at least about 1 million cells but in some situations up to 100 million over a period of 60-120 minutes. After 4 cycles of therapy tumor regression is noted.

One skilled in the art will appreciate that these methods, compositions, and cells are and may be adapted to carry out the objectives and obtain the ends and advantages mentioned, as well as those inherent therein. The methods, procedures, and devices described herein are presently representative of preferred embodiments and are exemplary and are not intended as limitations on the scope of the technology. Examples of such substitutions are non-natural enzymes and sugars. Those skilled in the art recognize that the aspects and embodiments set forth herein may be practiced separate from each other or in conjunction with each other. Therefore, combinations of separate embodiments are within the scope of the technology as disclosed herein. All patents and publications mentioned in the specification are indicative of the levels of those skilled in the art to which the technology pertains.

It is recognized that various modifications are possible within the scope of the embodiments disclosed. Thus, it should be understood that although the present technology has been specifically disclosed by preferred embodiments and optional features, modification and variation of the concepts herein disclosed may be resorted to by those skilled in the art, and that such modifications and variations are considered to be within the scope of this technology as defined by the disclosure.

## Claims

1. Fucosylated T cells for use in engraftment in a patient in need thereof by administering the fucosylated T cells to the patient, wherein the fucosylated T cells are obtained by:
(a) providing T cells from an *ex vivo* expanded cell population; and
(b) modifying said T cells by contacting said T cells with alpha 1,3-fucosyltransferase VI and/or alpha 1,3-fucosyltransferase VII to provide the fucosylated T cells.

2. A composition comprising an isolated population of fucosylated T cells obtained by steps (a) and (b) of claim 1 and a pharmaceutically-acceptable carrier.

3. The fucosylated T cells for use according to claim 1 or the composition of claim 2, wherein at least one of:
(a) the T cells are additionally contacted with a fucose carrier;
(b) the T cells are additionally contacted with a fucose carrier, and wherein the alpha 1,3-fucosyltransferase VI and/or alpha 1,3-fucosyltransferase VI is mixed together with said fucose carrier; and
(c) the T cells are additionally contacted with a fucose carrier, and wherein said fucose carrier is guanosine diphosphate fucose.

4. A method of enhancing homing and engraftment ability of T cells, the method comprising the steps of:
(a) providing T cells from an *ex vivo* expanded cell population; and
(b) modifying said T cells by contacting said T cells with alpha 1,3-fucosyltransferase VI and/or alpha 1,3-fucosyltransferase VII to provide fucosylated T cells.

5. The method of claim 4, further comprising at least one additional step selected from the group consisting of:
(a) contacting the T cells with a fucose carrier, wherein the alpha 1,3-fucosyltransferase is mixed together with said fucose carrier, and wherein said fucose carrier is guanosine diphosphate fucose; and
(b) combining said fucosylated T cells with a pharmaceutically-acceptable carrier to provide a composition capable of administration via a route selected from a group consisting of: intravenously, intraarterially, intramuscularly, subcutaneously, transdermally, intratracheally, intraperitoneally, intravitreally, into bone compartments, or into spinal fluid.

6. Fucosylated endothelial progenitor cells for use in engraftment in a patient in need thereof by administering the fucosylated endothelial progenitor cells to the patient, wherein the fucosylated endothelial progenitor cells are obtained by:
(a) providing endothelial progenitor cells from an *ex vivo* expanded cell population; and
(b) modifying said endothelial progenitor cells by contacting said endothelial progenitor cells with alpha 1,3-fucosyltransferase VI and/or alpha 1,3-fucosyltransferase VI to provide the fucosylated endothelial progenitor cells.

7. A composition comprising an isolated population of fucosylated endothelial progenitor cells obtained by steps (a) and (b) of claim 6 and a pharmaceutically-acceptable carrier.

8. The fucosylated endothelial progenitor cells for use according to claim 6 or the composition of claim 7, wherein at least one of:
(a) the endothelial progenitor cells are additionally contacted with a fucose carrier;
(b) the endothelial progenitor cells are additionally contacted with a fucose carrier, and wherein the alpha 1,3-fucosyltransferase VI and/or alpha 1,3-fucosyltransferase VI is mixed together with said fucose carrier; and
(c) the endothelial progenitor cells are additionally contacted with a fucose carrier, and wherein said fucose carrier is guanosine diphosphate fucose.

9. A method of enhancing homing and engraftment ability of endothelial progenitor cells, the method comprising the steps of:
(a) providing endothelial progenitor cells from an *ex vivo* expanded cell population; and
(b) modifying said endothelial progenitor cells by contacting said endothelial progenitor cells with alpha 1,3-fucosyltransferase VI and/or alpha 1,3-fucosyltransferase VI to provide the fucosylated endothelial progenitor cells.

10. The method of claim 9, further comprising at least one additional step selected from the group consisting of:
(a) contacting the endothelial progenitor cells with a fucose carrier, wherein the alpha 1,3-fucosyltransferase VI and/or alpha 1,3-fucosyltransferase VI is mixed together with said fucose carrier, and wherein said fucose carrier is guanosine diphosphate fucose; and
(b) combining said fucosylated endothelial progenitor cells with a pharmaceutically-acceptable carrier to provide a composition capable of administration via a route selected from a group consisting of: intravenously, intraarterially, intramuscularly, subcutaneously, transdermally, intratracheally, intraperitoneally, intravitreally, into bone compartments, or into spinal fluid.

## Patentansprüche

1. Fucosylierte T-Zellen zur Verwendung bei der Transplantation in einen dessen bedürftigen Patienten durch Verabreichung der fucosylierten T-Zellen an den Patienten, wobei die fucosylierten T-Zellen wie folgt erhalten werden:
(a) Bereitstellung von T-Zellen aus einer ex vivo expandierten Zellpopulation und
(b) Modifizieren der T-Zellen durch Inkontaktbringen der T-Zellen mit alpha-1,3-Fucosyltransferase VI und/oder alpha-1,3-Fucosyltransferase VII unter Bereitstellung der fucosylierten T-Zellen.

2. Zusammensetzung, umfassend eine isolierte Population von durch Schritte (a) und (b) von Anspruch 1 erhaltenen fucosylierten T-Zellen und einen pharmazeutisch unbedenklichen Träger.

3. Fucosylierte T-Zellen zur Verwendung nach Anspruch 1 oder Zusammensetzung nach Anspruch 2, wobei man mindestens eine der folgenden Handlungen durchführt:
(a) die T-Zellen werden zusätzlich mit einem Fucoseträger in Kontakt gebracht,
(b) die T-Zellen werden zusätzlich mit einem Fucoseträger in Kontakt gebracht, wobei die alpha-1,3-Fucosyltransferase VI und/oder alpha-1,3-Fucosyltransferase VII mit dem Fucoseträger gemischt wird, und
(c) die T-Zellen werden zusätzlich mit einem Fucoseträger in Kontakt gebracht, wobei es sich bei dem Fucoseträger um Guanosindiphosphatfucose handelt.

4. Verfahren zur Verbesserung der Zielfindungs- und Transplantierfähigkeit von T-Zellen, wobei das Verfahren die folgenden Schritte umfasst:
(a) die Bereitstellung von T-Zellen aus einer ex vivo expandierten Zellpopulation und
(b) das Modifizieren der T-Zellen durch Inkontaktbringen der T-Zellen mit alpha-1,3-Fucosyltransferase VI und/oder alpha-1,3-Fucosyltransferase VII unter Bereitstellung fucosylierter T-Zellen.

5. Verfahren nach Anspruch 4, welches weiterhin mindestens einen zusätzlichen Schritt ausgewählt aus der folgenden Gruppe umfasst:
(a) das Inkontaktbringen der T-Zellen mit einem Fucoseträger, wobei man die alpha-1,3-Fucosyltransferase mit dem Fucoseträger mischt und wobei es sich bei dem Fucoseträger um Guanosindiphosphatfucose handelt, und
(b) das Kombinieren der fucosylierten T-Zellen mit einem pharmazeutisch unbedenklichen Träger unter Bereitstellung einer für die Verabreichung über eine Route ausgewählt aus der Gruppe bestehend aus: intravenös, intraarteriell, intramuskulär, subkutan, transdermal, intratracheal, intraperitoneal, intravitreal, in Knochenkompartimente oder Rückenmarkflüssigkeit geeigneten Zusammensetzung.

6. Fucosylierte Endothelvorläuferzellen zur Verwendung bei der Transplantation in einen dessen bedürftigen Patienten durch Verabreichung der fucosylierten Endothelvorläuferzellen an den Patienten, wobei die fucosylierten Endothelvorläuferzellen wie folgt erhalten werden:
(a) Bereitstellung von Endothelvorläuferzellen aus einer ex vivo expandierten Zellpopulation und
(b) Modifizieren der Endothelvorläuferzellen durch Inkontaktbringen der Endothelvorläuferzellen mit alpha-1,3-Fucosyltransferase VI und/oder alpha-1,3-Fucosyltransferase VII unter Bereitstellung der fucosylierten Endothelvorläuferzellen.

7. Zusammensetzung, umfassend eine isolierte Population von durch Schritte (a) und (b) von Anspruch 6 erhaltenen fucosylierten Endothelvorläuferzellen und einen pharmazeutisch unbedenklichen Träger.

8. Fucosylierte Endothelvorläuferzellen zur Verwendung nach Anspruch 6 oder Zusammensetzung nach Anspruch 7, wobei man mindestens eine der folgenden Handlungen durchführt:
(a) die Endothelvorläuferzellen werden zusätzlich mit einem Fucoseträger in Kontakt gebracht,
(b) die Endothelvorläuferzellen werden zusätzlich mit einem Fucoseträger in Kontakt gebracht, wobei die alpha-1,3-Fucosyltransferase VI und/oder alpha-1,3-Fucosyltransferase VII mit dem Fucoseträger gemischt wird, und
(c) die Endothelvorläuferzellen werden zusätzlich mit einem Fucoseträger in Kontakt gebracht, wobei es sich bei dem Fucoseträger um Guanosindiphosphatfucose handelt.

9. Verfahren zur Verbesserung der Zielfindungs- und Transplantierfähigkeit von Endothelvorläuferzellen, wobei das Verfahren die folgenden Schritte umfasst:
(a) die Bereitstellung von Endothelvorläuferzellen aus einer ex vivo expandierten Zellpopulation und
(b) das Modifizieren der Endothelvorläuferzellen durch Inkontaktbringen der Endothelvorläuferzellen mit alpha-1,3-Fucosyltrans-ferase VI und/oder alpha-1,3-Fucosyl-transferase VII unter Bereitstellung der fucosylierten Endothelvorläuferzellen.

10. Verfahren nach Anspruch 9, welches weiterhin mindestens einen zusätzlichen Schritt ausgewählt aus der folgenden Gruppe umfasst:
(a) das Inkontaktbringen der Endothelvorläuferzellen mit einem Fucoseträger, wobei man die alpha-1,3-Fucosyltransferase VI und/oder alpha-1,3-Fucosyltransferase VII mit dem Fucoseträger mischt und wobei es sich bei dem Fucoseträger um Guanosindiphosphatfucose handelt, und
(b) das Kombinieren der fucosylierten Endothelvorläuferzellen mit einem pharmazeutisch unbedenklichen Träger unter Bereitstellung einer für die Verabreichung über eine Route ausgewählt aus der Gruppe bestehend aus: intravenös, intraarteriell, intramuskulär, subkutan, transdermal, intratracheal, intraperitoneal, intravitreal, in Knochenkompartimente oder Rückenmarkflüssigkeit geeigneten Zusammensetzung.

## Revendications

1. Lymphocytes T fucosylés pour utilisation dans le greffage chez un patient en ayant besoin par administration des lymphocytes T fucosylés au patient, les lymphocytes T fucosylés étant obtenus par :
(a) fourniture de lymphocytes T à partir d'une population de cellules expansées *ex vivo* ; et
(b) modification desdits lymphocytes T par mise en contact desdits lymphocytes T avec une alpha-1,3-fucosyltransférase VI et/ou alpha-1,3-fucosyltransférase VII pour produire les lymphocytes T fucosylés.

2. Composition comprenant une population isolée de lymphocytes T fucosylés obtenue par les étapes (a) et (b) de la revendication 1 et un véhicule pharmaceutiquement acceptable.

3. Lymphocytes T fucosylés pour utilisation selon la revendication 1 ou composition de la revendication 2, dans lesquels au moins l'un de:
(a) les lymphocytes T sont en outre mis en contact avec un support de fucose ;
(b) les lymphocytes T sont en outre mis en contact avec un support de fucose, et l'alpha-1,3-fucosyltransférase VI et/ou alpha-1,3-fucosyltransférase VII est mélangée conjointement avec ledit support de fucose ; et
(c) les lymphocytes T sont en outre mis en contact avec un support de fucose, et ledit support de fucose étant le guanosine diphosphate fucose.

4. Procédé d'augmentation de l'écotropisme et de la capacité de greffage de lymphocytes T, le procédé comprenant les étapes de :
(a) fourniture de lymphocytes T à partir d'une population de cellules expansées *ex vivo* ; et
(b) modification desdits lymphocytes T par mise en contact desdits lymphocytes T avec une alpha-1,3-fucosyltransférase VI et/ou alpha-1,3-fucosyltransférase VII pour obtenir des lymphocytes T fucosylés.

5. Procédé de la revendication 4, comprenant en outre au moins une étape additionnelle choisie dans le groupe constitué de :
(a) mise en contact des lymphocytes T avec un support de fucose, l'alpha-1,3-fucosyltransférase étant mélangé conjointement avec ledit support de fucose, et ledit support de fucose étant le guanosine diphosphate fucose ; et
(b) combinaison desdits lymphocytes T fucosylés avec un support pharmaceutiquement acceptable pour produire une composition capable d'administration par une voie choisie dans un groupe constitué de : la voie intraveineuse, la voie intra-artérielle, la voie intramusculaire, la voie sous-cutanée, la voie transdermique, la voie intratrachéale, la voie intrapéritonéale, la voie intravitréenne, dans des compartiments osseux, ou dans le fluide spinal.

6. Cellules progénitrices endothéliales fucosylées pour utilisation dans le greffage chez un patient en ayant besoin par administration des cellules progénitrices endothéliales fucosylées au patient, les cellules progénitrices endothéliales fucosylées étant obtenues par :
(a) fourniture de cellules progénitrices endothéliales à partir d'une population de cellules expansées *ex vivo* ; et
(b) modification desdites cellules progénitrices endothéliales par mise en contact desdites cellules progénitrices endothéliales avec une alpha-1,3-fucosyltransférase VI et/ou alpha-1,3-fucosyltransférase VII pour obtenir les cellules progénitrices endothéliales fucosylées.

7. Composition comprenant une population de cellules progénitrices endothéliales fucosylées isolée obtenue par les étapes (a) et (b) de la revendication 6 et un support pharmaceutiquement acceptable.

8. Cellules progénitrices endothéliales fucosylées pour utilisation selon la revendication 6 ou la composition de la revendication 7, dans laquelle au moins l'un de :
(a) les cellules progénitrices endothéliales sont en outre mises en contact avec un support de fucose ;
(b) les cellules progénitrices endothéliales sont en outre mises en contact avec un support de fucose, et l'alpha-1,3-fucosyltransférase VI et/ou alpha-1,3-fucosyltransférase VII étant mélangée conjointement avec ledit support de fucose ; et
(c) les cellules progénitrices endothéliales sont en outre mises en contact avec un support de fucose, et ledit support de fucose étant le guanosine diphosphate fucose.

9. Procédé d'amélioration de l'écotropisme et la capacité de greffage de cellules progénitrices endothéliales, le procédé comprenant les étapes de :
(a) fourniture de cellules progénitrices endothéliales à partir d'une population de cellules expansées *ex vivo* ; et
(b) modification desdites cellules progénitrices endothéliales par mise en contact desdites cellules progénitrices endothéliales avec une alpha-1,3-fucosyltransférase VI et/ou alpha-1,3-fucosyltransférase VII pour obtenir les cellules progénitrices endothéliales fucosylées.

10. Procédé de la revendication 9, comprenant en outre au moins une étape additionnelle choisie dans le groupe constitué de :
(a) mise en contact des cellules progénitrices endothéliales avec un support de fucose, dans laquelle l'alpha-1,3-fucosyltransférase VI et/ou alpha-1,3-fucosyltransférase VII est mélangée conjointement avec ledit support de fucose, et dans laquelle ledit support de fucose est le guanosine diphosphate fucose ; et
(b) combinaison desdites cellules progénitrices endothéliales fucosylées avec un support pharmaceutiquement acceptable pour obtenir une composition capable d'administration par une voie choisie dans un groupe constitué de : la voie intraveineuse, la voie intra-artérielle, la voie intramusculaire, la voie sous-cutanée, la voie transdermique, la voie intratrachéale, la voie intrapéritonéale, la voie intravitréenne, dans des compartiments osseux, ou dans le fluide spinal.
